# EUROPÄISCHE PATENTSCHRIFT

(11) **EP 2 559 427 B1**
(45) Veröffentlichungstag und Bekanntmachung des Hinweises auf die Patenterteilung: **24.09.2014**
(21) Anmeldenummer: 12006931.5
(22) Anmeldetag: 05.12.2008
(51) Int. Cl.: A61K 9/00, A61K 9/70

(54) **Transdermales therapeutisches System mit einem Gehalt an einem Modulator für nikotinische Acetylcholinrezeptoren (nAChR)**
Transdermal therapeutic system comprising a modulator for nicotinic acetylcholine receptors (nAChR)
Système thérapeutique transdermique contenant un modulateur de récepteurs nicotiniques de l'acétylcholine (nAChR)

(30) Priorität: 05.12.2007 DE 102007058504
(43) Veröffentlichungstag der Anmeldung: 20.02.2013
(62) Teilanmeldung aus: 08856066.9
(73) Patentinhaber: Acino AG, 83714 Miesbach (DE)
(72) Erfinder: Meyer, Elisabeth, 83714 Miesbach (DE); Tisa-Bostedt, Katalin, 82049 Pullach (DE); Beckert, Thomas, 88447 Birkenhard (DE)
(74) Vertreter: Forstmeyer, Dietmar

(56) Entgegenhaltungen:
- US-A1- 2006 128 676
- HIDEKI KAWAMATA ET AL: "Prediction of Plasma Concentration of GTS-21 in Hairless Rats Following Monolithic Transdermal Delivery", BIOLOGICAL & PHARMACEUTICAL BULLETIN, Bd. 25, Nr. 3, 1. Januar 2002 (2002-01-01) , Seiten 342-345, XP55049511, ISSN: 0918-6158, DOI: 10.1248/bpb.25.342

## Beschreibung

Die Erfindung betrifft ein transdermales therapeutisches System (TTS) mit einem Gehalt an mindestens einem Modulator für nikotinische Acetylcholinrezeptoren (nAChR-Modulator).

### Stand der Technik

Die transdermale Verabreichung von Wirkstoffen hat bestimmte Vorteile gegenüber herkömmlichen Verabreichungsverfahren, wie z.B. parenteraler oder oraler Verabreichung, die regelmäßig mit erheblichen Nebenwirkungen verbunden sind. So können beispielsweise manche Wirkstoffe nicht vom Verdauungstrakt aufgenommen werden und eine intravenöse oder subkutane Verabreichung mittels Injektion ist meist schmerzhaft und verletzend. Durch die transkutane Gabe der Wirkstoffe wird die gastrointestinale Passage und der damit verbundene First-pass-Metabolismus umgangen, was zu einer höheren Bioverfügbarkeit als unter oraler Medikation führt und die Nebenwirkungen parenteraler oder oraler Verabreichung vermeidet. Die mit TTS erreichbaren Plasmaspiegel sind in der Regel proportional zur jeweiligen Pflastergröße. Ähnlich einer intravenösen Dauerinfusion bleiben die Plasmaspiegel über die Zeit, in der das TTS mit der Haut in Kontakt ist, nahezu konstant. Um über einen längeren Zeitraum konstante Wirkstoffspiegel zu gewährleisten, müssen die TTS in regelmäßigen Abständen gewechselt werden, beispielsweise nach jeweils ca. 12 bis 168 Stunden.

Bisher finden vor allem Opioid-Wirkstoffe Anwendung bei der Behandlung von Schmerz. Der Einsatz von Opioid-Wirkstoffen birgt jedoch bekanntermaßen die Gefahr unerwünschter Arzneimittelwirkungen, wie beispielsweise Atemdepression sowie Ausbildung von psychischer und physischer Abhängigkeit. Des Weiteren kommt es bei chronischer Anwendung von Opioid-Wirkstoffen häufig zur Ausbildung von Toleranz, d.h. zu einer Wirkungsabschwächung infolge Gewöhnung an den Wirkstoff. Bei chronischer Anwendung von Opioid-Wirkstoffen ist es wegen Unverträglichkeit, Nebenwirkungen und zu kurzer oder schwacher Wirkung ferner oft notwendig, den Wirkstoff zu wechseln.

Die neue Wirkstoffklasse der Modulatoren für nikotinische Acetylcholinrezeptoren (im Folgenden auch als nAChR-Modulatoren bezeichnet) weist gegenüber den Opioid-Wirkstoffen ein breiteres therapeutisches Spektrum auf. So ist der Einsatz der Modulatoren für nikotinische Acetylcholinrezeptoren nicht auf den Einsatz als Analgetika beschränkt. Darüber hinaus wurden für die nAChR-Modulatoren bisher keine der vorstehend erwähnten unerwünschten Arzneimittelwirkungen beschrieben, wie sie bei den Opioid-Wirkstoffen auftreten.

Modulatoren für nikotinische Acetylcholinrezeptoren sind bekannt; vgl. u.a. Cassels et al., From ligand design to therapeutic efficacy: the challenge for nicotinic receptor research in Drug Discovery Today, 10 (2005) 1657-1665; Bannon et al. in J. Pharmacology Experimental Therapeutics, 285 (1998) 787-794; Wilens et al. in Am. J. Psychiatry, 156 (1999) 1931-1937 betreffend eine klinische Studie zur Behandlung von Erwachsenen mit Aufmerksamkeitsdefizit-Hyperaktivitätssyndrom (ADHD) mit einem transdermalen Pflaster mit dem Wirkstoff ABT-418 (37,5 oder 75 mg/Tag), wobei das/die Pflaster am Morgen von den Studienteilnehmern appliziert wurde(n) und vor dem Schlafengehen entfernt wurde(n); Daly et al. in Nat. Prod. Rep., 17 (2000) 131-135; Jain in Investigational Drugs, 5 (2004) 76-81. Derartige Modulatoren werden u.a. für die Behandlung von Schmerzen vorgeschlagen, vgl. etwa Cassels et al. loc. cit., oder zur Raucherentwöhnung, vgl. etwa die CHAMPIX-Tabletten des Handels mit Vareniclin-Tartrat als Wirkstoff. EP 0 950 057 Bl beschreibt 3-Pyridyl-Enantiomere, die in Standard-Schmerz-Tiermodellen eingesetzt wurden (loc. cit., Absatz [0071]). Aus DE 60 2004 003 252 T2 ist die Verwendung von 2H-[1,3]-Oxazino-[3,2]-indol-Derivaten zur Behandlung von neuropathischem Schmerz bekannt, wobei als geeignete Verabreichungsform u.a. Arzneistoffe enthaltende Pflaster vorgeschlagen werden. Dieses Dokument gibt jedoch keine Lehre, die Indolderivate als Modulatoren für nikotinische Acetylcholinrezeptoren zu verwenden. WO 2006/065233 und WO 2007/018738 beschreiben mit Bicycloheterocyclen substituierte Quinuclidin-Derivate als Modulatoren für nikotinische Acetylcholinrezeptoren. Einen Überblick über Modulatoren für nikotinische Acetylcholinrezeptoren und ihren Einsatz als Therapeutika kann ferner beispielsweise Arneric, SP et al., Neuronal nicotinic receptors: A perspective on two decades of drug discovery research in Biochemical Pharmacology 74 (2007) 1092-1101 entnommen werden.

HIDEKI KAWAMATA ET AL: "Prediction of Plasma Concentration of GTS-21 in Hairless Rats Following Monolithic Transdermal Delivery', BIOLOGICAL & PHARMACEUTICAL BULLETIN, Bd. 25, Nr. 3, 1. Januar 2002, beschreibt ein Transdermales therapeutisches System (TTS) zur Behandlung von Alzheimer-Krankheit, das GTS-21 (ein partieller Agonist am α7-Subtyp des nikotinischen Acetylcholin-Rezeptors) enthält. US 2006/128676 A1 beschreibt TTS, welche Agonisten am α7-Subtyp des nikotinischen Acetylcholin-Rezeptors enthalten.

### Beschreibung der Erfindung

Aufgabe der Erfindung ist es, eine Darreichungsform bereitzustellen, die die nicht tolerierbaren Nebenwirkungen bei oraler oder parenteraler Verabreichung von nAChR-Modulatoren wesentlich verringert oder gänzlich vermeidet. Insbesondere ist es eine Aufgabe der Erfindung ein transdermales therapeutische System (TTS) vorzusehen, mit dem sich mindestens ein Modulator für nikotinische Acetylcholinrezeptoren (nAChR-Modulator) transdermal applizieren lässt, vorzugsweise zur Behandlung von einer oder mehreren Krankheit(en), umfassend Schmerz, insbesondere neuropathischer Genese, neurodegenerative Erkrankungen, Schizophrenie, Aufmerksamkeitsdefizit-Hyperaktivitätssyndrom (ADHD), AAIM (age-associated memory impairment), insbesondere Alzheimer-Krankheit, und Raucherentwöhnung.

Die der Erfindung zugrunde liegende Aufgabe wird nun gelöst durch ein transdermales therapeutisches System (TTS) mit einem Gehalt an mindestens einem Modulator für nikotinische Acetylcholinrezeptoren (nAChR-Modulator). Weiterhin ist das erfindungsgemäße TTS vorzugsweise für eine Tragdauer von 24 bis 168 h ausgebildet und der eine nAChR-Modulator oder die mehreren nAChR-Modulatoren wird/werden vorzugsweise kontrolliert freigesetzt, wobei die Plateau-Konzentration des nAChR-Modulators/der nAChR-Modulatoren im Blutplasma (Tₘₐₓ) vorzugsweise 12 bis 96 h nach der Applikation des TTS erreicht wird. Dadurch kann in einer geringen therapeutischen Breite gearbeitet werden und die Bioverfügbarkeit an die Tragdauer angepasst werden.

Beipielsweise wird bei einem TTS, das für eine Tragdauer von 24 h ausgebildet ist, die Resorptionsgeschwindigkeit für den Wirkstoff, d.h. den mindestens einen nAChR-Modulator so eingestellt, dass die Plateau-Konzentration des Wirkstoffs im Blutplasma (Tₘₐₓ) 12 h nach der Applikation des TTS erreicht wird. Hingegen kann es bei einer längeren Tragdauer von z.B. 168 h vorteilhaft sein, wenn die Plateau-Konzentration des Wirkstoffs im Blutplasma (Tₘₐₓ) erst 96 h nach der Applikation des TTS erreicht wird.

Ferner bietet das erfindungsgemäße TTS etwa gegenüber einer oralen Verabreichung der nAChR-Modulatoren den Vorteil, dass die gastrointestinale Passage und der damit verbundene Firstpass-Metabolismus umgangen werden, bei dem für einzelne nAChR-Modulatoren toxische Nebenwirkungen beobachtet wurden.

Das erfindungsgemäße TTS umfasst:
(i) eine Deckschicht oder Abdeckschicht,
(ii) optional eine entfernbare Schutzschicht und
(iii-i) eine oder mehrere wirkstoffhaltige Schichten, die jeweils zwischen der Deckschicht und der optionalen entfernbaren Schutzschicht angeordnet sind, oder
(iii-ii) ein oder mehrere wirkstoffhaltige Reservoire, die jeweils zwischen der Deckschicht und der optionalen entfernbaren Schutzschicht angeordnet sind.

In der einen oder den mehreren wirkstoffhaltigen Schichten beziehungsweise in dem einen oder den mehreren wirkstoffhaltigen Reservoiren liegt der mindestens eine nAChR-Modulator jeweils sowohl gelöst oder teilweise gelöst als auch ungelöst vor. Weiterhin kann der mindestens eine nAChR-Modulator in der einen oder den mehreren wirkstoffhaltigen Schichten beziehungsweise in dem einen oder den mehreren wirkstoffhaltigen Reservoiren in dispergierter, teilweise dispergierter oder suspendierter Form vorliegen.

Ferner kann der mindestens eine nAChR-Modulator in der einen oder den mehreren wirkstoffhaltigen Schichten beziehungsweise in dem einen oder den mehreren wirkstoffhaltigen Reservoiren über einen Temperaturbereich von 2 °C bis zu 45 °C, vorzugsweise von 5 °C bis zu 40 °C, stärker bevorzugt von 8 °C bis zu 38 °C in gelöster oder teilweise gelöster Form vorliegen.

Ferner kann der mindestens eine nAChR-Modulator in der einen oder den mehreren wirkstoffhaltigen Schichten beziehungsweise in dem einen oder den mehreren wirkstoffhaltigen Reservoiren in einem Temperaturbereich von ca. 2 °C bis zu ca. 21 °C, z.B. während der Lagerung des erfindungsgemäßen TTS, in ungelöster Form vorliegen und in einem Temperaturbereich von ca. 25 °C bis zu ca. 37 °C, z.B. während des Tragens auf der Haut, in gelöster Form oder teilweise gelöster Form vorliegen. Hierdurch kann in vorteilhafter Weise eine verzögerte Freisetzung und damit eine langanhaltende und konstante Abgabe des mindestens einen nAChR-Modulators erzielt werden. Ohne auf eine Theorie festgelegt werden zu wollen, wird vermutet, dass durch die allmähliche Erwärmung des erfindungsgemäßen TTS nach dem Aufbringen auf die Haut nach und nach der mindestens eine nAChR-Modulator gelöst oder zumindest teilweise gelöst wird. Hierdurch kann beispielsweise das erfindungsgemäße TTS für eine Tragdauer von 24 bis 168 h ausgebildet werden und der mindestens eine nAChR-Modulator kontrolliert freigesetzt werden, wobei die Plateau-Konzentration des mindestens einen nAChR-Modulators im Blutplasma (Tₘₐₓ) 12 bis 96 h nach der Applikation des TTS erreicht wird.

Andere Möglichkeiten, wie das erfindungsgemäße TTS für eine Tragdauer von 24 bis 168 h ausgebildet werden kann und der mindestens eine nAChR-Modulator kontrolliert freigesetzt werden kann, wobei die Plateau-Konzentration des mindestens einen nAChR-Modulators im Blutplasma (Tₘₐₓ) 12 bis 96 h nach der Applikation des TTS erreicht wird, sind ebenfalls möglich, zum Beispiel durch die gezielte Auswahl der weiteren Bestandteile des erfindungsgemäßen TTS, insbesondere Kleber, Lösungsmittel, Permeationsförderer, Resorptionsmodulator etc.

Der Ausdruck "teilweise gelöst" beziehungsweise "teilweise dispergiert" bedeutet, dass mindestens 5 Gew.-%, bevorzugt mindestens 20 Gew.-%, stärker bevorzugt mindestens 40 Gew.-%, noch stärker bevorzugt mindestens 50 Gew.-%, noch stärker bevorzugt mindestens 60 Gew.-%, noch stärker bevorzugt mindestens 80 Gew.-%, am stärksten bevorzugt mindestens 95 Gew.-% des Gesamtgehalts des mindestens einen nAChR-Modulators in gelöster beziehungsweise dispergierter Form vorliegt.

Wenn der mindestens eine nAChR-Modulator nicht vollständig gelöst vorliegt, kann er eine mittlere Partikelgröße (X50-Wert) von 1 bis 200 µm, vorzugsweise 2 bis 100 µm, stärker bervorzugt 5 bis 50 µm aufweisen.

Das erfindungsgemäße TTS kann zur Behandlung von einer oder mehreren Krankheit(en), umfassend Schmerz, kognitive Defizite, neurodegenerative Erkrankungen, Schizophrenie, Aufmerksamkeitsdefizit-Hyperaktivitätssyndrom (ADHD), AAIM (age-associated memory impairment), insbesondere Alzheimer-Krankheit, und Raucherentwöhnung vorgesehen werden.

Der mit dem erfindungsgemäßen TTS zu behandelnde Schmerz umfasst insbesondere post-operative Schmerzen, Schmerzen als Begleiterscheinung einer Krebserkrankung, chronische Schmerzen des Skelettsystems oder der Muskulatur, idiopathische Schmerzen, Schmerzen neuropathischer Genese, hervorgerufen insbesondere durch diabetische Neuropathie, AIDS oder Krebs.

Die mit dem erfindungsgemäßen TTS zu behandelnden kognitiven Defizite stehen insbesondere in Zusammenhang mit bzw. werden hervorgerufen durch Alzheimer-Krankheit und/oder Schizophrenie.

Weitere mit dem erfindungsgemäßen TTS zu behandelnde Krankheiten umfassen insbesondere Parkinson, Angsterkrankungen, Depression, Tourette-Syndrom, Colitis ulcerosa.

Bei dem erfindungsgemäßen TTS ist der mindestens eine Modulator für nAChR ein Agonist oder ein partieller Agonist am α4ß2-Subtyp des nikotinischen Acetylcholin-Rezeptors, ausgewählt aus der Gruppe von MEM-3454, MEM-63908, TC-5619, AR-R-17779, PNU-282987 und SSR 180711.

Für die Behandlung von kognitiven Defiziten sind insbesondere erfindungsgemäße TTS geeignet, die mindestens einen Agonisten oder partiellen Agonisten am α7-Subtyp des nikotinischen Acetylcholin-Rezeptors enthalten. So sind insbesondere erfindungsgemäße TTS vorteilhaft für die Behandlung von kognitiven Defiziten, die mindestens einen Modulator für nAChR enthalten, der ausgewählt wurde aus der Gruppe von MEM-3454, MEM-63908, TC-5619, AR-R-17779, PNU-282987 und SSR 180711.

Ferner kann es sich bei dem erfindungsgemäßen TTS der mindestens eine Modulator für nAChR als pharmazeutisch verträgliches Salz vorgesehen sein.

Ferner kann das erfindungsgemäße TTS zusätzlich einen α3β4-Rezeptor Antagonisten enthalten, insbesondere Mecamylamin.

Das erfindungsgemäße TTS kann 0,1 bis 1000 mg, insbesondere 1 bis 500 mg, vorzugsweise 1 bis 250 mg Modulator(en) für nAChR enthalten.

Ferner kann das erfindungsgemäße TTS gekennzeichnet sein durch eine Abgaberate von 10 bis 250 µg/h und insbesondere 15 bis 150 µg/h Modulator(en) für nAChR.

Ferner kann das erfindungsgemäße TTS gekennzeichnet sein durch eine Abgaberate von 0,5 bis 10 µg/cm² h Modulator(en) für nAChR.

Das erfindungsgemäße TTS mit den ein oder mehreren wirkstoffhaltigen Schichten kann ein Matrix-System sein, insbesondere vom monolithischen Typ "drug-in-adhesive patch".

Das erfindungsgemäße TTS mit den ein oder mehreren wirkstoffhaltigen Reservoiren kann ein Reservoirsystem sein.

Bei dem erfindungsgemäßen TTS kann der mindestens eine Modulator für nAChR in einer oder in mehreren wirkstoffhaltigen Schichten oder in einem oder in mehreren wirkstoffhaltigen Reservoiren enthalten sein.

Bei dem erfindungsgemäßen TTS kann der mindestens eine Modulator für nAChR in der (den) wirkstoffhaltigen Schicht(en) oder in dem (den) wirkstoffhaltigen Reservoir(en) als Dispersion vorliegen.

Unter Dispersion werden insbesondere folgende Systeme verstanden:
- molekulardispers:: sogenannte "wahre" Lösungen; Moleküle sind im Lösungsmittel dispergiert / gelöst. Teilchendurchmesser < 1 nm.
- kolloiddispers:: Feststoffteilchen sind im Lösungsmittel dispergiert. Teilchendurchmesser zwischen 1 und 500 nm.
- grobdispers:: Teilchen im Lösungsmittel > 500 nm.

Ferner kann bei dem erfindungsgemäßen TTS der mindestens eine Modulator für nAChR in einem Reservoir enthalten sein.

Bei dem erfindungsgemäßen TTS kann das Reservoir den mindestens einen Modulator für nAChR als Lösung in einem Lösungsmittel umfassen.

Ferner kann bei dem erfindungsgemäßen TTS das Reservoir den mindestens einen Modulator für nAChR als Lösung in einem organischen Lösungsmittel umfassen, insbesondere Ethanol.

Bei dem erfindungsgemäßen TTS kann der Modulator für nAChR in der wirkstoffhaltigen Schicht oder in den wirkstoffhaltigen Schichten molekulardispers vorliegen.

Ferner kann das TTS hautseitig eine Klebschicht oder eine von der Abdeckschicht getragene Klebschicht aufweisen.

Bei dem erfindungsgemäßen TTS kann die Klebschicht eine selbstklebende Polymermatrixschicht sein.

Ferner kann bei dem erfindungsgemäßen TTS die selbstklebende Polymermatrixschicht eine wirkstoffhaltige Schicht mit einem Gehalt an Modulator für nAChR sein, insbesondere eine Schicht gemäß (iii-i).

Ferner kann bei dem erfindungsgemäßen TTS die Klebschicht zumindest eine Komponente umfassen oder aus einer Komponente bestehen, die aus der Gruppe ausgewählt ist von natürlichem Kautschuk, synthetischem Kautschuk, Polyacrylat, Polyvinylacetat, Polyisobutylen, Silikon, insbesondere Polydimethylsiloxan, und Hydrogel, insbesondere hochmolekularem Polyvinylpyrrolidon, Polyvinylalkohol und oligomerem Polyethylenoxid oder Mischungen derselben.

Insbesondere kann bei dem erfindungsgemäßen TTS die Klebschicht ein Polyacrylat umfassen oder daraus bestehen.

Bei dem erfindungsgemäßen TTS kann das Polyacrylat ein oder mehrere Monomer-Einheiten von einem oder mehreren Monomeren umfassen, die aus der Gruppe ausgewählt sind von n-Butylacrylat, iso-Butylacrylat, Propylacrylat, Methylacrylat, 2-Ethylhexylacrylat, 2-Hydroxy-ethylacrylat und 2-Hydroxyethylmethacrylat.

Bei dem erfindungsgemäßen TTS kann es sich bei dem synthetischen Kautschuk um ein Styrol-Butadien-Styrol-Block-Copolymer oder ein Styrol-Butadien-Block-Copolymer handeln.

Ferner kann (können) bei dem erfindungsgemäßen TTS die wirkstoffhaltige(n) Schicht(en) und/oder die Klebschicht ein Vernetzungsmittel enthalten.

Ferner kann es sich bei dem erfindungsgemäßen TTS bei der wirkstoffhaltigen Schicht um eine Matrix aus einem Material handeln, das durch Hautfeuchtigkeit an- oder auflösbar ist.

Ferner kann bei dem erfindungsgemäßen TTS die Matrix ein Material umfassen, das durch Hautfeuchtigkeit an- oder auflösbar ist.

Ferner kann das erfindungsgemäße TTS den mindestens einen Modulator für nAChR in einem Hydrogel umfassen.

Das erfindungsgemäße TTS kann gekennzeichnet sein durch Hydroxymethylcellulose, Hydroxypropylcellulose, Carbopol oder Polvinylalkohol als Hydrogel.

Ferner kann bei dem erfindungsgemäßen TTS der mindestens eine Modulator für nAChR als Dispersion oder als Suspension in einem nicht-wassermischbaren Polymer vorliegen.

Ferner kann bei dem erfindungsgemäßen TTS der mindestens eine Modulator für nAChR als Dispersion oder als Suspension in einem Hydrogel vorliegen.

Ferner kann das erfindungsgemäße TTS ein Verdickungsmitel oder Quellmittel umfassen, vorzugsweise Hydroxypropylcellulose, insbesondere Klucel.

Ferner kann es sich bei dem erfindungsgemäßen TTS um ein Elektrodenpflaster mit ein oder mehreren hautnahen Elektroden und mit ein oder mehreren deckschichtnahen Gegenelektroden handeln. Ein derartiges System ist beispielsweise EP 99 920 628.7 und EP 03 005 664.2 zu entnehmen.

Gemäß einer weiteren Ausführungsform kann die der Erfindung zugrunde liegende Aufgabe auch durch ein Iontophorese-Pflaster gelöst werden. Relevanter Stand der Technik für iontophoretische Systeme sind DE 37 03 321, WO 87/04936, WO 91/16077, WO 92/04938, WO 00/53256, US 3 991 755, US 4 141 359, US 5 415 629 und US 5 944 685.

Ferner kann das erfindungsgemäße TTS als weitere Schicht eine Steuermembran aufweisen.

Ferner kann das erfindungsgemäße TTS hautseitig als weitere Schicht eine Silikonschicht aufweisen.

Ferner kann bei dem erfindungsgemäßen TTS die Steuermembran auf der hautabgewandten Seite der Klebschicht vorgesehen sein.

Ferner kann das erfindungsgemäße TTS ein oder mehrere Permeationsförderer enthalten.

So kann das erfindungsgemäße TTS mindestens einen Permeationsförderer aus der Gruppe von Ethylalkohol, Isopropylalkohol, Octylphenol, Polyethylenglykol (PEG), insbesondere PEG400, Propylenglykol, Polyethylenglykoloctylphenylether, Ölsäure, Linolsäure, Linolensäure, Sojaöl, Aloe Vera Extrakt, Dodecanol, Harnstoff, Labrafil, Labrasol, Fettsäureester, insbesondere Isopropylmyristat, Monolaurat, vorzugsweise Methyllaurat, Propylenglykolmonolaurat, Glycerolmonolaurat und Polyethylenglykolmonolaurat, Octyldodecyllactat, Glycerolmonooleat und Glykolmonooleat, Transcutol, Tocopherol, N-Decylmethylsulfoxid, Triacetin und N-Methylpyrrolidon umfassen.

Ferner kann das erfindungsgemäße TTS ein oder mehrere Konservierungsmittel enthalten, insbesondere aus der Gruppe von Alkoholen, quaternären Aminen, organischen Säuren, Parabenen und Phenolen.

Ferner kann das erfindungsgemäße TTS ein oder mehrere Übersättigungsstabilisatoren enthalten, z. B. Polyvinylpyrrolidon.

Ferner kann das erfindungsgemäße TTS einen oder mehrere Resorptionsmodulator(en) enthalten; d.h. einen Hilfsstoff, der die Abgabe des Wirkstoffs aus dem TTS fördert oder verzögert, wodurch die Aufnahme des Wirkstoffs durch die Haut weiter gesteuert werden kann.

So kann das erfindungsgemäße TTS mindestens einen Resorptionsmodulator aus der Gruppe von Isovaleriansäure, Caprylsäure, Bernsteinsäure, Maleinsäure, Glutarsäure, Adipinsäure, Pimelinsäure und Zitronensäure umfassen.

Ferner kann das erfindungsgemäße TTS gekennzeichnet sein durch eine Abdeckfolie, bestehend aus oder umfassend ein Material aus der Gruppe von Polyolefin, Polyester, Polyvinylidenchlorid, Polyurethan, Baumwolle, Viskose oder Wolle. Vorzugsweise besteht die Deckfolie aus Polyester.

Dabei kann das erfindungsgemäße TTS gekennzeichnet sein durch eine Abdeckfolie einer Stärke von 0,01 bis 1,5 und insbesondere 0,03 bis 1,0 mm.

Ferner kann das erfindungsgemäße TTS eine Fläche von 2 bis 100 cm², bevorzugt 5 bis 50 cm², insbesondere 10 bis 40 cm², beispielsweise etwa 20 cm² aufweisen.

Schließlich kann das erfindungsgemäße TTS mit einem Overtape versehen sein.

Nachstehend wird auf folgende Referenzbeispiele Bezug genommen.

### Referenzbiespiel 1 (Matrix-Pflaster)

Für die Herstellung einer Matrix wird Epibatidin (5 bis 15 Gew.-%) in Ethylmethylketon (Verhältnis 1 : 4,2) gelöst und Aloe Vera Extrakt als transdermales Penetrationshilfsmittel (0,1 bis 20 Gew.-%) und ein Styrol-Butadien-Styrol-Polymer (Durotak DT-6173) zugegeben und das Ganze vermischt, bis es homogen ist. Das homogene Gemisch wird danach mit einem mittleren Flächengewicht von etwa 50 g/m² mit einem Handbeschichtungsgerät auf einer Polyesterfolie (Abdeckfolie) aufgebracht. Die beschichtete Folie wird etwa 15 min lang bei etwa 80°C getrocknet, um das Lösungsmittel Ethylmethylketon zu verdampfen. Schließlich wird noch eine silikonisierte Abziehfolie aufkaschiert.

### Referenzbeispiel 2 (Matrix-Pflaster)

Analog zu Beispiel 1 wird eine Matrix hergestellt, wobei anstelle von Epibatidin ABT-594 in Ethanol und zur Herstellung einer Klebstoffschicht bzw. druckempfindlichen Haftschicht Duro-Tak 2051 verwendet wird. Die Klebstoffschicht wird mit Hilfe einer geeigneten Beschichtungseinrichtung (beispielsweise reverse roll coater) auf eine silikonisierte Trägerfolie (aus beispielsweise Polyester und insbesondere Polyterephthalsäureester) derart aufgetragen, daß ein Flächengewicht des getrockneten Klebstoffs von 80 g/m² resultiert.

### Referenzbeispiel 3 (Hydrogel-Pflaster)

Das Reservoir ist ein wirkstoffhaltiges Hydrogel. Für die Herstellung wird ABT-894 unter Erwärmen in einem protischen Lösungsmittel dispergiert. Der Gelbildner Hydroxypropylcellulose wird anschließend in einer finalen Konzentration von 2 - 4 % zugesetzt. Das Hydrogel verbleibt über Nacht zur Quellung.

Auf einen physikalisch vernetzten, geschlossenzelligen Polyolefinschaumstoff mit der Dicke von z.B. 1 mm wird beidseitig eine druckempfindliche Haftkleberschicht aufgebracht.

Aus diesem Polyolefinschaum wird ein kreisflächenförmiges Stück mit einem Durchmesser von z.B. 26 mm ausgestanzt. Aus diesem Stück wird mittig ein z.B. 1 cm² großer Kreis ausgestanzt, so dass ein Ring entsteht. In diesen Ring wird das wirkstoffhaltige Hydrogel eingegeben.

Eine Polyester Trägerfolie wird auf die der Haut abgewandten Seite des Reservoirs in Verbindung mit der mit einem Haftkleber beschichteten Polyolefinring aufgebracht. Auf die ebenfalls Klebstoffbeschichtete untere, der Trägerfolie abgewandten Fläche wird eine Polyethylenmembran geklebt. Im Anschluß wird auf die Membran eine mit einem druckempfindlichen Silikonkleber beschichtete Schutzfolie aufgebracht. Die Schutzfolie wird vor dem Aufkleben des Systems auf die Haut entfernt.

### Referenzbeispiel 4 (Elektroden-Pflaster)

Auf einen physikalisch vernetzten, geschlossenzelligen Polyolefinschaumstoff mit der Dicke von z.B. 1 mm wird beidseitig eine druckempfindliche Haftschicht aufgebracht. Aus diesem Polyolefinschaum wird ein kreisflächenförmiges Stück mit einem Durchmesser von z.B. 26 mm ausgestanzt. Aus diesem Stück wird mittig ein z.B. 1 cm² großer Kreis ausgestanzt, so dass ein Ring entsteht. Dieser Ring bildet den Hohlraum für das wirkstoffhaltige Reservoir.

ABT-089 wird analog zu Beispiel 3 als ionischer Arzneistoff in einer Reservoirmatrix dispergiert und in die Öffnung des Elektrodenpflasters eingebracht. Die Konzentration des Wirkstoffs im Reservoir beträgt 1 %.

Als Elektrodenmaterial wird ein glattes Edelstahlquadratmaschengewebe mit definierter Maschenweite und definiertem Drahtdurchmesser verwendet. In einem Galvanisierungsbad wird das Gitter mit einer Schicht aus Edelmetall überzogen.

Mit Hilfe einer Stanzform werden kreisförmige Elektroden mit einem Durchmesser von 16 mm ausgestanzt. Jede Elektrode hat einen seitlich verlängerten Streifen, der über den Rand des Pflasters ragt und die Möglichkeit bietet, eine externe Spannungsquelle anzuschließen. Beidseitig wird das Reservoir durch das Elektrodenpaar begrenzt. Die Elektroden haften mit Hilfe des druckempfindlichen Silikonklebers an dem Schaumstoff.

Die voneinander isolierten Zuleitungen werden jeweils individuell mit einer Spannungsquelle verbunden, so dass die zur Trägerfolie zugewandte Elektrode als Anode und die der Haut zugewandte Elektrode als Kathode geschaltet werden kann. Eine Trägerfolie, die einseitig mit einem selbstklebenden Haftkleber beschichtet ist, wird auf die der Haut abgewandten Elektrode geklebt, um das Elektrodenpaar und das Reservoir zu schützen.

Auf die untere, der Trägerfolie (= Deckschicht) abgewandten Elektrode wird eine mit einem druckempfindlichen, hydrophoben Silikonkleber beschichtete Schutzfolie aufgebracht. Die Schutzfolie wird vor dem Aufkleben des Systems auf die Haut entfernt.

Durch Anlegen einer einheitlichen Spannung, mit Hilfe einer externen Spannungsquelle im Niedrigspannungsbereich, migriert der protonierte Wirkstoff an die hautzugewandte Elektrode.

## Patentansprüche

1. Transdermales therapeutisches System (TTS) umfassend
(i) eine Deckschicht,
(ii) optional eine entfernbare Schutzschicht und
(iii-i) eine oder mehrere wirkstoffhaltige Schichten, die jeweils zwischen der Deckschicht und der optionalen entfernbaren Schutzschicht angeordnet sind und mindestens einen nAChR-Modulator in gelöster, teilweise gelöster oder ungelöster Form enthalten, oder
(iii-ii) ein oder mehrere wirkstoffhaltige Reservoire, die jeweils zwischen der Deckschicht und der optionalen entfernbaren Schutzschicht angeordnet sind und mindestens einen nAChR-Modulator in gelöster, teilweise gelöster oder ungelöster Form enthalten,
wobei der mindestens eine Modulator für nAChR ein Agonist oder partieller Agonist am α7-Subtyp des nikotinischen Acetylcholin-Rezeptors ist, ausgewählt aus der Gruppe von MEM-3454, MEM-63908, TC-5619, AR-R-17779, PNU-282987 und SSR 180711.

2. TTS nach Anspruch 1 zur Behandlung von einer oder mehreren Krankheit(en), umfassend Schmerz, kognitive Defizite, neurodegenerative Erkrankungen, Schizophrenie, Aufmerksamkeitsdefizit-Hyperaktivitätssyndrom (ADHD), AAIM (age-associated memory impairment), insbesondere Alzheimer-Krankheit, Raucherentwöhnung, Parkinson, Angsterkrankungen, Depression, Tourette-Syndrom, Colitis ulcerosa.

3. TTS nach mindestens einem der vorhergehenden Ansprüche, wobei der mindestens eine Modulator für nAChR als pharmazeutisch verträgliches Salz vorgesehen ist.

4. TTS nach mindestens einem der vorhergehenden Ansprüche, wobei das TTS zusätzlich einen α3β4-Rezeptor-Antagonisten enthält, insbesondere Mecamylamin.

5. TTS nach mindestens einem der vorhergehenden Ansprüche, wobei der mindestens eine Modulator für nAChR in einer oder in mehreren wirkstoffhaltigen Schichten oder in einem oder in mehreren wirkstoffhaltigen Reservoiren enthalten ist.

6. TTS nach mindestens einem der vorhergehenden Ansprüche, wobei das TTS hautseitig eine Klebschicht oder eine von der Deckschicht getragene Klebschicht aufweist.

7. TTS nach mindestens einem der vorhergehenden Ansprüche, wobei das TTS den mindestens einen Modulator für nAChR in einem Hydrogel umfasst.

8. TTS nach mindestens einem der vorhergehenden Ansprüche, wobei das TTS ein Verdickungsmitel oder Quellmittel umfasst, vorzugsweise Hydroxypropylcellulose.

9. TTS nach mindestens einem der vorhergehenden Ansprüche, wobei das TTS ein Elektrodenpflaster mit ein oder mehreren hautnahen Elektroden und mit ein oder mehreren deckschichtnahen Gegenelektroden ist, oder wobei das System ein Iontophorese-Pflaster ist.

10. TTS nach mindestens einem der vorhergehenden Ansprüche, wobei das TTS als weitere Schicht eine Steuermembran aufweist.

11. TTS nach mindestens einem der vorhergehenden Ansprüche, wobei das TTS hautseitig als weitere Schicht eine Silikonschicht aufweist.

12. TTS nach mindestens einem der vorhergehenden Ansprüche, wobei das TTS einen oder mehrere Permeationsförderer enthält.

13. TTS nach mindestens einem der vorhergehenden Ansprüche, wobei das TTS einen oder mehrere Übersättigungsstabilisatoren enthält.

14. TTS nach mindestens einem der vorhergehenden Ansprüche, wobei das TTS einen oder mehrere Resorptionsmodulator(en) enthält.

15. Verwendung mindestens eines Modulators, wie in Anspruch 1 definiert, zur Herstellung eines transdermalen therapeutischen Systems (TTS) gemäß Anspruch 1.

## Claims

1. Transdermal therapeutic system (TTS) comprising
(i) a backing layer,
(ii) optionally, a removable protecting layer and
(iii-i) one or more active-ingredient-containing layers, each of which is arranged between the backing layer and the optional removable protecting layer and comprises at least one nAChR modulator in dissolved, partially dissolved or undissolved form, or
(iii-ii) one or more active-ingredient-containing reservoirs, each of which is arranged between the backing layer and the optional removable protecting layer and comprises at least one nAChR modulator in dissolved, partially dissolved or undissolved form,
wherein the at least one nAChR modulator is an agonist or partial agonist of the α7 subtype of the nicotinic acetylcholine receptor, selected from the group MEM-3454, MEM-63908, TC-5619, AR-R-17779, PNU-282987 and SSR 180711.

2. TTS according to claim 1 for the treatment of one or more disease(s) including pain, cognitive deficits, neurodegenerative diseases, schizophrenia, attention-deficit hyperactivity syndrome (ADHD), AAMI (age-associated memory impairment), especially Alzheimer's disease, smoker withdrawal, Parkinson's, anxiety disorders, depression, Tourette's syndrome, ulcerative colitis.

3. TTS according to at least one of the preceding claims, wherein the at least one nAChR modulator is provided in the form of a pharmaceutically acceptable salt.

4. TTS according to at least one of the preceding claims, wherein the TTS additionally comprises a α3β4-receptor antagonist, especially mecamylamine.

5. TTS according to at least one of the preceding claims, wherein the at least one nAChR modulator is contained in one or more active-ingredient-containing layers or in one or more active-ingredient-containing reservoirs.

6. TTS according to at least one of the preceding claims, wherein the TTS has, on the skin side, an adhesive layer or an adhesive layer supported by the backing layer.

7. TTS according to at least one of the preceding claims, wherein the TTS comprises the at least one nAChR modulator in a hydrogel.

8. TTS according to at least one of the preceding claims, wherein the TTS comprises a thickening agent or swelling agent, preferably hydroxypropylcellulose.

9. TTS according to at least one of the preceding claims, wherein the TTS is an electrode patch having one or more electrodes in proximity of the skin and one or more counter-electrodes in proximity of the backing layer, or wherein the system is an iontophoresis patch.

10. TTS according to at least one of the preceding claims, wherein the TTS has a control membrane as a further layer.

11. TTS according to at least one of the preceding claims, wherein the TTS has, on the skin side, a silicone layer as a further layer.

12. TTS according to at least one of the preceding claims, wherein the TTS comprises one or more permeation promoters.

13. TTS according to at least one of the preceding claims, wherein the TTS comprises one or more supersaturation stabilisers.

14. TTS according to at least one of the preceding claims, wherein the TTS comprises one or more absorption modulator(s).

15. Use of at least one modulator, as defined in claim 1, for the preparation of a transdermal therapeutic system (TTS) according to claim 1.

## Revendications

1. Système thérapeutique transdermique (STT) comprenant
(i) une couche de revêtement,
(ii) en option, une couche de protection pouvant être enlevée, et
(iii-i) une ou plusieurs couches contenant de la substance active, qui sont respectivement disposées entre la couche de revêtement et la couche de protection optionnelle pouvant être enlevée et qui contiennent au moins un modulateur nAChR sous la forme soluble, partiellement soluble ou non solubilisée, ou
(iii-ii) un ou plusieurs réservoirs contenant de la substance active qui sont respectivement disposés entre la couche de revêtement et la couche de protection optionnelle pouvant être enlevée et qui contiennent au moins un modulateur nAChR sous la forme soluble, partiellement soluble ou non solubilisée,
où au moins un modulateur pour nAChR est un agoniste ou un agoniste partiel sur le sous-type α7 du récepteur nicotinique acétylcholine, choisi dans le groupe constitué par le MEM-3454, le MEM-63908, le TC-5619, l'AR-R-17779, le PNU-282987 et le SSR 180711.

2. STT selon la revendication 1 pour le traitement d'une ou de plusieurs maladie(s), comportant de la douleur, des déficits cognitifs, des maladies neurodégénératives, la schizophrénie, le syndrome du déficit d'attention et d'hyperactivité (SDAHA), l'AAIM (pertes de mémoire associées à l'âge), en particulier la maladie d'Alzheimer, la désaccoutumance au tabac, la maladie de Parkinson, les angoisses maladives, la dépression, le syndrome de la Tourette, la colite ulcéreuse.

3. STT selon au moins l'une des revendications précédentes dans lequel un modulateur pour nAChR est prévu sous la forme d'un sel pharmaceutiquement acceptable.

4. STT selon au moins l'une des revendications précédentes dans lequel le STT contient en outre un antagoniste récepteur α3β4, en particulier, de la mécamylamine.

5. STT selon au moins l'une des revendications précédentes dans lequel au moins un modulateur pour nAChR est contenu dans une ou dans plusieurs couches contenant de la substance active, ou dans un ou dans plusieurs réservoirs contenant de la substance active.

6. STT selon au moins l'une des revendications précédentes dans lequel le STT présente une couche adhésive du côté de la peau, ou une couche adhésive supportée par la couche de revêtement.

7. STT selon au moins l'une des revendications précédentes dans lequel le STT comprend au moins un modulateur pour nAChR dans un hydrogel.

8. STT selon au moins l'une des revendications précédentes dans lequel le STT comprend un agent d'épaississement ou un agent gonflant, de préférence de l'hydroxypropylcellulose.

9. STT selon au moins l'une des revendications précédentes dans lequel le STT est un pansement à électrodes comprenant une ou plusieurs électrodes proches de la peau et comprenant une ou plusieurs contre-électrodes proches de la couche de revêtement, ou dans lequel le système est un pansement d'iontophorèse.

10. STT selon au moins l'une des revendications précédentes dans lequel le STT présente une membrane de réglage en tant que couche supplémentaire.

11. STT selon au moins l'une des revendications précédentes dans lequel le STT présente une couche de silicone en tant que couche supplémentaire du côté de la peau.

12. STT selon au moins l'une des revendications précédentes, dans lequel le STT contient un ou plusieurs promoteurs de perméation.

13. STT selon au moins l'une des revendications précédentes dans lequel le STT contient un ou plusieurs stabilisants de sursaturation.

14. STT selon au moins l'une des revendications précédentes dans lequel le STT contient un ou plusieurs modulateur(s) de résorption.

15. Utilisation d'au moins un modulateur, tel que défini dans la revendication 1 pour la fabrication d'un système thérapeutique transdermique (STT) selon la revendication 1.
